(19) Europäisches Patentamt
European Patent Office
Office européen des brevets

(11) Publication number: **0 322 800 A1**

(12) **EUROPEAN PATENT APPLICATION**

(21) Application number: **88121599.0**

(51) Int. Cl.4: **C07D 249/08 , C07D 233/60**

(22) Date of filing: **23.12.88**

(30) Priority: **25.12.87 JP 331470/87**

(43) Date of publication of application: **05.07.89 Bulletin 89/27**

(84) Designated Contracting States: **AT BE CH DE ES FR GB GR IT LI NL SE**

(71) Applicant: **SUMITOMO PHARMACEUTICALS COMPANY, LIMITED**
**2-8, Doshomachi 2-chome, Chuo-ku**
**Osaka-shi Osaka-fu(JP)**

(72) Inventor: **Ohashi, Naohito**
**1-325, Kuwata-cho 2-chome**
**Ibaraki-shi, Osaka-fu(JP)**
Inventor: **Fujimoto, Koji**
**29-7, Ooike 2-chome**
**Ibaraki-shi Osaka-fu(JP)**
Inventor: **Mori, Kazuo**
**51, Minoazayamagata**
**Kawanishi-shi, Hyogo-ken(JP)**
Inventor: **Tanaka, Yoshihiro**
**177-1-607, Kibe-cho**
**Ikeda-shi, Osaka-fu(JP)**
Inventor: **Saji, Ikutaro**
**8-Q-401, Aobaokaminami**
**Suita-shi, Osaka-fu(JP)**
Inventor: **Nishizawa, Toshio**
**6-36, Maeda-cho**
**Niihama-shi, Ehime-ken(JP)**

(74) Representative: **Vossius & Partner**
**Siebertstrasse 4 P.O. Box 86 07 67**
**D-8000 München 86(DE)**

(54) **Process for preparation of sulfone derivatives.**

(57) A process for preparing sulfone derivatives of the formula:

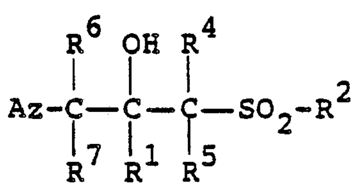

(I)

wherein $R^1$ and $R^2$ are each a hydrogen atom, a lower alkyl group or a substituted or unsubstituted cyclo(lower)alkyl, cyclo(lower)alkylmethyl, lower alkenyl, heterocyclic, aryl or ar(lower)alkyl group, $R^4$, $R^5$, $R^6$ and $R^7$ are each a hydrogen atom, a lower alkyl group, a lower alkenyl group or a substituted or unsubstituted aryl grcup and Az is a 1,2,4- or 1,3,4-triazole or imidazole ring, which comprises subjecting a sulfide or sulfoxide compound of the formula:

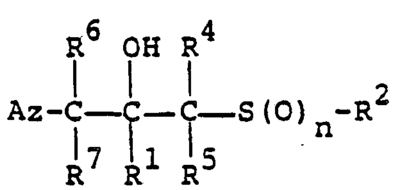

(II)

EP 0 322 800 A1

wherein $R^1$, $R^2$, $R^4$, $R^5$, $R^6$, $R^7$ and Az are each as defined above and n is an integer of 0 or 1 to oxidation with an oxidizing agent in the presence of a metal catalyst.

## PROCESS FOR PREPARATION OF SULFONE DERIVATIVES

The present invention relates to a process for preparation of sulfone derivatives. More particularly, it relates to an industrially advantageous process for preparation of sulfone derivatives.

Sulfone derivatives of the formula:

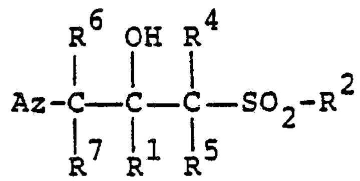

(I)

(wherein $R^1$ and $R^2$ are each a hydrogen atom or a lower alkyl group, a substituted or unsubstituted cyclo(lower)alkyl, cyclo(lower)alkylmethyl, lower alkenyl, heterocyclic, aryl or ar(lower)alkyl group, $R^4$, $R^5$, $R^6$ and $R^7$ are each a hydrogen atom, a lower alkyl group, a lower alkenyl group or a substituted or unsubstituted aryl group and Az is a 1,2,4-or 1,3,4-triazole or imidazole ring) are known to be useful as antimicrobial agents, particularly antifungal agents.

In the above significances and throughout the specification, the term "lower" is intended to mean a group having not more than 8 carbon atoms, particularly not more than 6 carbon atoms. The term "lower alkyl" covers a straight or branched alkyl group of not more than 6 carbon atoms, preferably from 1 to 4 carbon atoms (e.g. methyl, ethyl, propyl, isopropyl, butyl, sec-butyl, isobutyl, t-butyl). The term "lower alkenyl" covers an alkenyl group of not more than 6 carbon atoms, preferably from 2 to 4 carbon atoms (e.g. vinyl, allyl, 2-butenyl). The term "cyclo(lower)alkyl" covers a cycloalkyl group of not more than 6 carbon atoms, preferably from 3 to 6 carbon atoms (e.g. cyclopropyl, cyclopentyl, cyclohexyl). The term "aryl" means an aromatic hydrocarbon group having not more than 20 carbon atoms, preferably a phenyl group. As the halogen atom, there may be exemplified fluorine, chlorine, bromine, etc. The term "lower alkoxy" covers an alkoxy group having not more than 6 carbon atoms, preferably from 1 to 4 carbon atoms (e.g. methoxy, ethoxy) and the term "halo(lower)alkyl" includes a haloalkyl group having not more than 6 carbon atoms, preferably from 1 to 4 carbon atoms (e.g. trifluoromethyl, 1,1,2,2-tetrafluoroethyl). The term "halo(lower)alkoxy" includes a haloalkoxy group having not more than 6 carbon atoms, favorably from 1 to 4 carbon atoms (e.g. trifluoromethoxy, 1,1,2,2-tetrafluoroethoxy). The term "heterocyclic" covers a heterocyclic group having not more than 8 ring atoms, of which at least one is a hetero atom (e.g. nitrogen, oxygen, sulfur), and its specific examples are pyridyl, furyl, thienyl, etc. The substituent which may be present on the cyclo(lower)alkyl group, the cyclo(lower)alkylmethyl group, the lower alkenyl group, the heterocyclic group, the aryl group or the ar(lower)alkyl group may be halogen, nitro, lower alkyl, halo(lower)-alkyl, lower alkoxy, halo(lower)alkoxy, phenyl, halophenyl, phenoxy, benzyl, benzyloxy, etc. Thus, specific examples of the substituted aryl or ar(lower)alkyl group are as follows: alpha-methylbenzyl, 2-, 3- or 4-chlorophenyl, 2,4- or 2,6-dichlorophenyl, 2-, 3- or 4-fluorophenyl, 2,4-or 2,6-difluorophenyl, 2-, 3- or 4-bromophenyl, 2-chloro-4-fluorophenyl, 2-fluoro-4-chlorophenyl, 2-chloro-6-fluorophenyl, 2-, 3- or 4-methoxyphenyl, 2,4-dimethoxyphenyl, 2-, 3- or 4-ethoxyphenyl, 2-, 3- or 4-nitrophenyl, 2-chloro-5-nitrophenyl, 2-, 3- or 4-methylphenyl, 2,4-dimethylphenyl, 2-, 3- or 4-t-butylphenyl, 2-, 3- or 4-trifluoromethylphenyl, 2-, 3- or 4-trifluoromethoxyphenyl, 2-, 3- or 4-(1,1,2,2-tetrafluoroethyl)phenyl, 2-fluoro-4-methoxyphenyl, 2-methoxy-4-fluorophenyl, 2-methoxy-4-chlorophenyl, 2-methoxy-4-fluorophenyl, 2-, 3- or 4-phenoxyphenyl, 2-, 3- or 4-phenylphenyl, 2-, 3- or 4-benzylphenyl, 2-, 3- or 4-benzyloxyphenyl, 2-, 3- or 4-(4-chlorobenzyloxy)phenyl, 2-, 3- or 4-(4-fluorobenzyloxy)phenyl, etc.

The sulfone derivatives (I) have heretofore been produced by subjecting the corresponding sulfide or sulfoxide compounds of the formula:

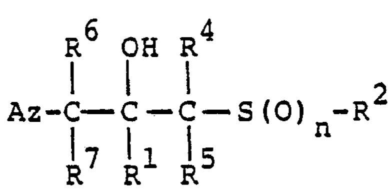

(II)

wherein $R^1$, $R^2$, $R^4$, $R^5$, $R^6$, $R^7$ and Az are each as defined above and n is an integer of 0 or 1 to oxidation with m-chloroperbenzoic acid (EP-A-061835, EP-A-095828, EP-A-178533). However, this oxidation procedure is not suitable for adoption at an industrial scale, because m-chloroperbenzoic acid as the oxidizing agent is expensive and has a problem in safety.

On the other hand, it is known that sulfides are generally oxidized with an oxidizing agent such as hydrogen oxide in the presence of a metal catalyst such as tungstenic acid to give the corresponding sulfones (J. Org. Chem., 28, 1140 (1960)). However, this oxidation procedure can usually oxidize amines under the same conditions as above into their N-oxides (EP-A-201934). Because of this reason, said oxidation procedure has been considered to be not suitable for production of the sulfone derivatives (I) from the corresponding sulfide or sulfoxide compounds (II) which have both a sulfide or sulfoxide structure and an amine structure such as a triazole or an imidazole in their molecules.

As the result of an extensive study seeking an advantageous process for production of the sulfone derivatives (I), it has now been unexpectedly found that the sulfide or sulfoxide compounds (II) can be oxidized with an oxidizing agent in the presence of a metal oxide to give the sulfone derivatives (I) without any material by-production of the corresponding N-oxides. In comparison with said conventional process for oxidiation of the sulfide or sulfoxide compounds (II) with m-chloroperbenzoic acid, the new process is much more suitable for industrial adoption, because the reagents as used therein are less expensive and very safe.

According to the process of the invention, the sulfide or sulfoxide compound (II) is reacted with an oxidizing agent in the presence of a metal catalyst to give the sulfone derivative (I).

As the metal catalyst, there may be used any substance comprising a metal ion, a metal oxide or a metal salt (e.g. ammonium salt, alkali metal salt, alkaline earth metal salt, silver salt). Taking easy availability, facility on handling and reactivity into consideration, preferred examples of the metal catalyst are tungstic acid or its salts (e.g. sodium tungstate, potassium tungstate, ammonium tungstate, calcium tungstate), tungstophosphoric acid or its salts (e.g. sodium tungstophosphate, ammonium tungstophosphate), molybdic acid or its salts (e.g. ammonium molybdate, potassium molybdate, calcium molybdate, sodium molybdate); phosphomolybdic acid or its salts (e.g. ammonium phosphomolybdate, sodium phosphomolybdate), vanadic acid or its salts (e.g. silver vanadate, sodium vanadate, ammonium vanadate), metavanadic acid or its salts (e.g. ammonium metavanadate), titanium compounds (e.g. titanium oxyacetylacetate, titanium tetrapropoxide, titanium tetra-n-butoxide, titanium tetraisopropoxide), etc.

As the oxidizing agent, there may be used hydrogen peroxide, organic peroxides, sodium hypochlorite, sodium chlorate, sodium perchlorate, lithium perchlorate, etc. Among them, hydrogen peroxide or sodium hypochlorite in an aqueous solution is espectially favorable in low cost and high safety.

A typical procedure for carrying out the process of the invention will be hereinafter explained in detail without limiting the scope of the invention thereto.

The process is usually carried out by admixing the sulfide or sulfoxide compound (II) with an appropriate inert solvent and a metal catalyst and then adding an oxidizing agent thereto, whereby the reaction proceeds to give the sulfone derivative (I). While the addition of the oxidizing agent at the last is favorable for performing the reaction safely, this is not essential. In other words, mixation of the starting material, the inert solvent, the metal catalyst and the oxidizing agent may be effected in any optional order.

As the inert solvent, there may be used water, alcohols (e.g. methanol, ethanol, isopropanol, ethylene glycol), ethers (tetrahydrofuran, dimethoxyethane), esters (e.g. ethyl acetate), aromatic hydrocarbons (e.g. toluene, xylene), non-aromatic hydrocarbons (e.g. hexane, cyclohexane, heptane), halogenated hydrocarbons (e.g. chloroform, dichloromethane, dichloroethane, chlorobenzene), ketones (e.g. acetoe, methylethylketone), dimethylformamide, etc. Their mixtures are also usable. When organic solvents are used, there may frequently happen that the metal catayst is not dissolved. In such case, addition of water to make a mixed solvent is preferred. It is also preferred to add a phase transfer catalyst such as a quarternary ammonium salt to the mixed solvent when it is not evenly miscible and remains as the two phase mixture.

The metal catalyst may be used in an amount as optionally decided depending on the amount of the sulfide or sulfoxide compound (II) and its amount is usually from about 0.0005 to 1 mol to one mol of the sulfide or sulfoxide compound (II). The oxidizing agent is generally used in an amount of about 0.7 to 50 mol to one mol of the sulfide or sulfoxide compound (II). The amount of the inert solvent may be normally from about 2 to 500 parts by weight to one part by weight of the sulfide or sulfoxide compound (II), but it is not a critical factor.

The reaction proceeds normally at a temperature of about -20°C to the boiling temperature of the solvent, preferably from room temperature to the boiling temperature of the solvent.

Recovery of the thus produced sulfone derivative (I) from the reaction mixture may be accomplished by a per se conventional procedure. For instance, the reaction mixture is treated with sodium thiosulfate or the

like to decompose the oxidizing agent remaining therein and adjusted to pH 4 to 10, followed by collection of the precipitated crystals. When the reaction mixture includes an organic solvent, the organic solvent is removed therefrom, water is added to the residue, and the precipitated crystals are collected. In case of the crystals being not precipitated, the residual mixture is extracted with a halogenated hydrocarbon (e.g. chloroform, dichloromethane) to obtain the sulfone derivative (I).

The sulfone derivative (I) as well as the sulfide or sulfoxide compound (II) have at least one asymmetric center and include various isomers, all of which should be regarded to be within the scope of the invention.

The sulfoxide compound (II: n = 1) is obtainable from the corresponding sulfide compound (II: n = 0) by a known method, and the latter (II: n = 0) can be obtained by reacting an epoxide compound of the formula:

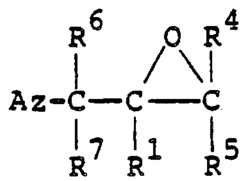

(III)

wherein $R^1$, $R^4$, $R^5$, $R^6$, $R^7$ and Az are each as defined above with a metal compound of the formula:

$R^2S^{\ominus}M^{\oplus}$ (IV)

wherein $R^2$ is as defined above and M is an alkali metal in an inert solvent (EP-A-061835). In this reaction, there is usually obtained the reaction mixture containing not only the desired sulfide compound (II: n = 0) but also the metal compound (IV) as unreacted and/or the disulfide compound as by-produced; elimination of these undesired compounds is thus necessary for isolation of the sulfide compound (II: n = 0). On this occasion, however, any appropriate means must be applied in order to prevent an offensive odor producing from the reaction mixture.

Advantageously, the oxidation in the process of this invention can be carried out successfully with said reaction mixture comprising the sulfide compound (II: n =0), i.e. without elimination of the undesired compounds, whereby the sulfide compound (II: n = 0) is converted into the sulfone derivative (I) and simultaneously the contami nating metal and/or disulfide compounds are converted into the corresponding sulfinic or sulfonic acid derivatives. Since these sulfinic or sulfonic acid derivatives do not have any offensive odor, their elimination from the reaction mixture does not require application of any prevention means.

In case of the application of the process of this invention to the reaction mixture comprising the sulfide compound (II: n = 0) as above, a metal catalyst and an oxidizing agent as exemplified above may be added, if necessary, together with an inert solvent as exemplified above to the reaction mixture, whereby the oxidation is effected in one pot omitting the operation for isolation of the sulfide compound (II: n = 0) and avoiding the treatment of the reaction mixture having an offensive odor.

In the above reaction of the epoxide compound (III) with the metal compound (IV) giving the sulfide compound (II: n = 0), it is desirable not to use dimethylsulfoxide, which is quite popular in the reaction of this kind. This is because the use of dimethylsulfoxide in the above reaction naturally results in its presence at the subsequent oxidation where dimethylsulfoxide is oxidized with the oxidizing agent to dimethylsulfone, thereby the oxidizing agent being wasted. Further, the amount of the oxidizing agent is to be somewhat increased, because not only the sulfide compound (II: n = 0) but also the starting metal compound (IV) and the by-produced disulfide compound are to be oxidized.

Practical and presently preferred embodiments of the process of the invention are illustratively shown in the following examples.

Example 1

(±)-threo-2-(2,4-Difluorophenyl)-3-methylsulfonyl-1-(1,2,4-triazol-1-yl)butan-2-ol:-

To a solution of (±)-threo-2-(2,4-difluorophenyl)-3-methylthio-1-(1,2,4-triazol-1-yl)butan-2-ol (0.50 g; 1.67

mM), water (1.5 g) and methanol (15 g), sodium tungstate dihydrate (11 mg; 0.033 mM) was added at room temperature, followed by dropwise addition of 31 % aqueous hydrogen peroxide (0.46 g; 4.19 mM) at 55° C. The resultant mixture was stirred at 60° C for 6 hours. The reaction mixture was cooled to room temperature and combined with 5 % sodium thiosulfate (0.38 g). After removal of methanol (13.4 g) by distillation at 50 to 60° C, water (1.5 g) was added thereto. The resulting mixture was cooled and stirred at a temperature below 5° C for 2 hours. The precipitated crystals were collected by filtration, washed with water and dried under reduced pressure to give (±)-threo-2-(2,4-difluorophenyl)-3-methylsulfonyl-1-(1,2,4- triazol-1-yl)butan-2-ol (0.49 g). m.p., 211 - 212° C.

Example 2

(±)-threo-2-(2,4-Difluorophenyl)-3-methylsulfonyl-1-(1,2,4-triazol-1-yl)butan-2-ol:-

To a solution of (±)-erythro-2-[(1,2,4-triazol-1-yl)methyl]-2-(2,4-difluorophenyl)-3-methyloxirane (3.5 g; 13.9 mM) in methanol (10.5 g), 15 % aqueous solution of methylmercaptan sodium (9.76 g; 20.9 mM) was added at room temperature under nitrogen stream, and the resultant mixture was heated to 60° C and stirred for 4 hours. The reaction mixture was cooled to room temperature and, after addition of methanol (87.5 g), adjusted to pH 8.6 with aqueous hydrochloric acid. An aqueous solution (0.8 g) containing sodium tungstate dihydrate (92 mg; 0.28 mM) was added to the mixture, which was heated to a temperature of 55 to 60° C. 31 % Aqueous hydrogen peroxide (4.60 g; 41.9 mM) was dropwise added thereto, followed by stirring for 3 hours. The reaction mixture was cooled to room temperature, 5 % sodium thiosulfate (4.3 g) was added thereto and the pH was adjusted to 9.1 with 5 % aqueous sodium hydrogen oxide. Methanol (87.5 g) was removed therefrom by distillation at a temperature of 50 to 60° C, and water (10.5 g) was added thereto, followed by cooling. The reaction mixture was stirred at a temperature below 5° C for 3 hours. The precipitated crystals were collected by filtration, washed with water and dried under reduced pressure to give (±)-threo-2-(2,4-difluorophenyl)-3-methylsulfonyl-1-(1,2,4-triazol-1-yl)butan-2-ol (4.03 g). Yield, 87.3 %. m.p., 211 - 212° C.

Example 3

(±)-threo-2-(2,4-Difluorophenyl)-3-methylsulfonyl-1-(1,2,4-triazol-1-yl)butan-2-ol:-

To a solution of (±)-threo-2-(2,4-difluorophenyl)-3-methylthio-1-(1,2,4-triazol-1-yl)butan-2-ol (0.50 g), water (1.5 g) and methanol (15 g), ammonium matavanadate (10 mg) was added at room temperature. The resultant mixture was adjuted to pH 4 with dilute hydrochloric acid. 31 % Aqueous hydrogen peroxide (0.46 g) was dropwise added thereto at 55° C, followed by stirring at 60° C for 5 hours. Stirring was continued for additional 10 hours, during which 31 % aquoues hydrogen peroxide (0.84 g in total) was added thereto every several hour. The reaction mixture was cooled to room temperature and adjusted to pH 9.2 with 5 % aqueous sodium hydroxide (0.67 g). Methanol (11.9 g) was removed therefrom at a temperature of 50 to 60° C, and water (1.5 g) was added thereto, followed by cooling. After stirring at a temperature below 5° C for 2 hours, the precipitated crystals were collected by filtration, washed with water and dried under reduced pressure to give (±)-threo-2-(2,4-difluorophenyl)-3-methylsulfonyl-1-(1,2,4-triazol-1-yl)butan-2-ol (0.42 g).

Example 4

(±)-threo-2-(2,4-Difluorophenyl)-3-methylsulfonyl-1-(1,2,4-triazol-1-yl)butan-2-ol:-

To a solution of (±)-threo-2-(2,4-difluorophenyl)-3-methylthio-1-(1,2,4-triazol-1-yl)butan-2-ol (0.50 g), water (1.5 g) and methanol (15 g), sodium molybdate dihydrate (20 mg) was added at room temperature, and 31 % aqueous hydrogen peroxide (0.46 g) was dropwise added thereto at 55° C, followed by stirring at 60° C. After 4 hours, 31 % aquoues hydrogen peroxide (0.46 g) was added thereto, and 2 hours thereafter, 31 % aquoues hydrogen peroxide (0.46 g) was again added thereto while stirring for further 2 hours.

Methanol (12.8 g) was removed therefrom by distillation at a temperature of 50 to 60 °C, and water (1.5 g) was added thereto, followed by cooling. The reaction mixture was stirred at a temperature below 5 °C for 2 hours, and the precipitated crystals were collected by filtration, washed with water and dried under reduced pressure to give (±)-threo-2-(2,4-difluorophenyl)-3-methylsulfonyl-1-(1,2,4-triazol-1-yl)butan-2-ol (0.50 g).

Example 5

(±)-threo-2-(2,4-Dichlorophenyl)-3-methylsulfonyl-1-(1,2,4-triazol-1-yl)butan-2-ol:-

To a solution of (±)-threo-2-(2,4-dichlorophenyl)-3 methylthio-1-(1,2,4-triazol-1-yl)butan-2-ol (0.50 g), water (1.5 g) and methanol (15 g), sodium tungstate dihydrate (25 mg) was added at room temperature, and 31 % aqueous hydrogen peroxide (0.41 g) was dropwise added thereto at 55 °C, followed by stirring at 60 °C for 3 hours. The reaction mixture was cooled to room temperature, followed by addition of 5 % sodium thiosulfate (0.27 g). Methanol (14 g) was removed from the reaction mixture by distillation at a temperature of 50 to 60 °C, and water (1.5 g) was added thereto, followed by cooling. The reaction mixture was stirred at a temperature below 5 °C for 2 hours, and the precipitated crystals were collected by filtration, washed with water and dried under reduced pressure to give (±)-threo-2-(2,4-dichlorophenyl)-3-methylsulfonyl-1-(1,2,4-triazol-1-yl)butan-2-ol (0.503 g). m.p., 178 - 182 °C.

Example 6

(±)-threo-2-(2,4-Dichlorophenyl)-3-pentylsulfonyl-1-(1,2,4-triazol-1-yl)butan-2-ol:-

To a solution of (±)-threo-2-(2,4-dichlorophenyl)-3-pentylthio-1-(1,2,4-triazol-1-yl)butan-2-ol (0.50 g), water (1.5 g) and methanol (15 g), sodium tungstate dihydrate (21 mg) was added at room temperature, and 31 % aqueous hydrogen peroxide (0.35 g) was dropwise added thereto at 55 °C, followed by stirring at 60 °C for 3 hours. The reaction mixture was cooled to room temperature, followed by addition of 5 % sodium thiosulfate (0.18 g). Methanol (15 g) was removed from the reaction mixture by distillation at a temperature of 50 to 60 °C, and the reaction mixture was extracted with a mixture of chloroform (20 g) and water (20 g). The aqueous layer was extracted with chloroform (10 g), the chloroform extract was combined with the chloroform layer and the combined mixture was washed with water (20 g). The chloroform layer was concentrated and dried under reduced pressure to give (±)-threo-2-(2,4-dichlorophenyl)-3-pentylsu lfonyl-1-(1,2,4-triazol-1-yl)butan-2-ol (0.528 g). m.p., 106 - 109 °C.

Example 7

(±)-threo-2-(2,4-Difluorophenyl)-3-methylsulfonyl-1-(1,2,4-triazol-1-yl)butan-2-ol:-

To a solution of (±)-threo-2-(2,4-difluorophenyl)-3-methylthio-1-(1,2,4-triazol-1-yl)butan-2-ol (0.20 g), water (0.6 g) and methanol (6 g), titanium tetraisopropoxide (24 mg) was added at room temperature, and 31 % aqueous hydrogen peroxide (0.22 g) was dropwise added thereto at 55 °C, followed by stirring at 60 °C for 3.5 hours. Methanol (20 g) was added thereto, and the reaction mixture was stirred under reflux for 20 minutes and filtered. The filtrate was concentrated and dried. Methanol (0.6 g) and water (0.6 g) were added thereto, followed by heating. The heated mixture was cooled and stirred at a temperature below 5 °C for 2 hours. The precipitate crystals were collected by filtration, washed with water and dried under reduced pressure to give (±)-threo-2-(2,4-difluorophenyl)-3-methylsulfonyl-1-(1,2,4-triazol-1-yl)butan-2-ol (0.20 g).

Example 8

(±)-threo-2-(2,4-Difluorophenyl)-3-methylsulfonyl-1-(1,2,4-triazol-1-yl)butan-2-ol:

To a solution of (±)-threo-2-(2,4-difluorophenyl)-3-methylthio-1-(1,2,4-triazol-1-yl)butan-2-ol (0.20 g) and dichloroethane (3.0 g), titanium tetraisopropoxide (24 mg) was added at room temperature. Following the same procedure as in Example 7, there was obtained (±)-threo-2-(2,4-difluorophenyl)-3-methylsulfonyl-1-(1,2,4-triazol-1-yl)butan-2-ol (0.19 g).

Example 9

(±)-threo-2-(2,4-Difluorophenyl)-3-methylsulfonyl-1-(1,2,4-triazol-1-yl)butan-2-ol:-

To a solution of (±)-threo-2-(2,4-difluorophenyl)-3-methylthio-1-(1,2,4-triazol-1-yl)butan-2-ol (20.0 g; 66.8 mmol) in methanol (100 g), sodium tungstate dihydrate (110 mg; 0.33 mmol) was added at room temperature. The resultant mixture was adjusted to a pH of less than 1 with 35 % aqueous hydrochloric acid, and 35 % aqueous hydrogen peroxide (15.6 g; 160.3 mmol) was dropwise added thereto, followed by stirring at 60 °C for 2 hours. The reaction mixture was cooled to room temperature, and after addition of 5 % sodium thiosulfate (33.2 g), adjusted to pH 8.5 with 10 % aqueous sodium hydroxide (40.0 g), followed by stirring at a temperature below 5 °C for 4 hours. The precipitated crystals were collected by filtration, washed with water (33 g x 2) and cold methanol (33 g) and dried under reduced pressure to give (±)-threo-2-(2,4-difluorophenyl)-3-methylsulfonyl-1-(1,2,4-triazol-1-yl)butan-2-ol (21.0 g) as crude crystals. m.p., 211 - 212 °C. Recrystallization from methanol gave the objective product. m.p., 211 - 212 °C.

## Claims

1. A process for preparing sulfone derivatives of the formula:

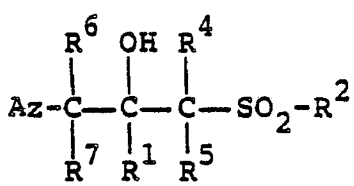

(I)

wherein $R^1$ and $R^2$ are each a hydrogen atom, a lower alkyl group or a substituted or unsubstituted cyclo(lower)alkyl, cyclo(lower)alkylmethyl, lower alkenyl, heterocyclic, aryl or ar(lower)alkyl group, $R^4$, $R^5$, $R^6$ and $R^7$ are each a hydrogen atom, a lower alkyl group, a lower alkenyl group or a substituted or unsubstituted aryl group and Az is a 1,2,4- or 1,3,4-triazole or imidazole ring, which comprises subjecting a sulfide or sulfoxide compound of the formula:

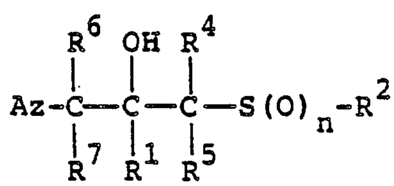

(II)

wherein $R^1$, $R^2$, $R^4$, $R^5$, $R^6$, $R^7$ and Az are each as defined above and n is an integer of 0 or 1 to oxidation with an oxidizing agent in the presence of a metal catalyst.

2. The process according to claim 1, whrein $R^1$ is 2,4-difluorophenyl, $R^2$ is methyl, either one of $R^4$ or $R^5$ is methyl and the other is hydrogen and Az is 1,2,4-triazole.

3. The process according to claim 1, wherein the catalyst is tungstic acid and the oxidizing agent is hydrogen peroxide.

4. The process according to claim 1, wherein the solvent is methanol or aqueous methanol.

European Patent Office

# EUROPEAN SEARCH REPORT

Application number

EP 88121599.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 97, no. 9, August 30, 1982, Columbus, Ohio, USA<br>NIPPON KAYAKU CO., LTD. "Nitrophenyl β-hydroxyethyl sulfones" page 604, column 1, abstract-no. 72 060v<br>& Jpn. Kokai Tokkyo Koho JP 82 48,962<br>-- | 1 | C 07 D 249/08<br>C 07 D 233/60 |
| Y | CHEMICAL ABSTRACTS, vol. 91, no. 9, August 27, 1979, Columbus, Ohio, USA<br>KOZHEVNIKOV, I. V.; et al. "Liquid-phase oxidation of alkyl sulfides catalyzed by a heteropoly acid" page 537, column 1, abstract-no. 73 935x<br>& Kinet. Katal. 1979, 20(2), 506-10<br>-- | 1 | |
| Y | CHEMICAL ABSTRACTS, vol. 66, no. 9, February 27, 1967, Columbus, Ohio, USA<br>L. KUHNEN "Oxidation of sulfoxides with hydroperoxides" page 3 562, column 1, abstract-no. 37 567d<br>& Angew. Chem. 78(20), 937(1966), Angew. Chem. Intern. Ed. Eng. 5(10), 893(1966)<br>---- | 1 | TECHNICAL FIELDS SEARCHED (Int. Cl.4)<br>C 07 D 249/00<br>C 07 D 233/00 |

The present search report has been drawn up for all claims

| Place of search | Date of completion of the search | Examiner |
|---|---|---|
| VIENNA | 10-03-1989 | HAMMER |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number

-2-

EP 88121599.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 72, no. 2, January 12, 1970, Columbus, Ohio, USA<br>HARDY, FREDERICK E.; et al. "Vanadium pentoxide-catalyzed oxidation of thio-compounds with hydrogen peroxide"<br>page 255, column 2, abstract-no. 6 560r<br>& J. Chem. Soc. C 1969, (17), 2334-6<br>-- | 1 | |
| P,Y | CHEMICAL ABSTRACTS, vol. 108, no. 17, April 25, 1988, Columbus, Ohio, USA<br>TAKANO, JINKO "Triazole derivatives as agrochemical fungicides"<br>page 277, column 2, abstract-no. 145 444f<br>& Jpn. Kokai Tokkyo Koho JP 62,265,205<br>-- | 1 | TECHNICAL FIELDS SEARCHED (Int. Cl.4) |
| Y | CHEMICAL ABSTRACTS, vol. 103, no. 17, October 28, 1985, Columbus, Ohio, USA<br>SUMITOMO CHEMICAL CO., LTD. "N-Substituted triazole derivatives"<br>page 725, column 1, abstract-no. 141 972a<br>& Jpn. Kokai Tokkyo Koho JP 60 69,071<br>-- | 1 | |

The present search report has been drawn up for all claims

| Place of search | Date of completion of the search | Examiner |
|---|---|---|
| VIENNA | 10-03-1989 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

-3-
EP 88121599.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 100, no. 11, March 12, 1984, Columbus, Ohio, USA<br>PFIZER CORP. "Fungicidal triazoles" page 547, column 2, abstract-no. 85 706r<br>& Jpn. Kokai Tokkyo Koho JP 58,185,571<br>-- | 1 | |
| Y | EP - A1 - 0 178 533 (SUMITOMO)<br>* Page 4, line 27 - page 5, line 11; examples 6,9,15, 18,31,30,34,37 *<br>-- | 1 | |
| D,A | EP - A1 - 0 061 835 (JCJ)<br>* Example 4; table I; examples 27-31 *<br>-- | 1 | TECHNICAL FIELDS SEARCHED (Int. Cl.4) |
| A | EP - A2 - 0 180 136 (BAYER)<br>* Page 29, lines 9-21; example 4, table I; examples 73, 94-100, 127, 132, 144, 152-154, 156<br>-- | 1 | |
| D,A | EP - A1 - 0 095 828 (PFIZER)<br>* Page 8, line 16 - page 10, line 4, examples 19, 24, 25 *<br>-- | 1 | |

The present search report has been drawn up for all claims

| Place of search | Date of completion of the search | Examiner |
|---|---|---|
| VIENNA | 10-03-1988 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number



EP 88121599.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - A1 - 3 402 166 (HOECHST) * Page 30, lines 14-27; example 9 * -- | 1 | |
| A | DE - A1 - 3 200 414 (BAYER) * Example 2 * ---- | 1 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 10-03-1989 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82